# EUROPEAN PATENT APPLICATION

(11) **EP 3 815 680 A1**
(43) Date of publication of application: **05.05.2021**
(21) Application number: 19824521.9
(22) Date of filing: 25.06.2019
(51) Int. Cl.: A61K 9/16, A61K 9/20, A61K 31/167, A61K 31/192, A61K 31/197, A61K 31/63, A61K 47/02, A61K 47/04, A61K 47/10, A61K 47/26, A61K 47/32, A61K 47/34, A61K 47/36, A61K 47/38

(54) **PROCESSING METHOD FOR RAW DRUG PARTICLES OF NON-UNIFORM PARTICLE SIZE**

(30) Priority: 26.06.2018 JP 2018121227
(71) Applicant: Nippon Zoki Pharmaceutical Co., Ltd., Chuo-ku Osaka-shi, Osaka 541-0046 (JP)
(72) Inventor: SAKAMOTO, Hiroshi, Sakai-shi, Osaka 590-0134 (JP); KOMAI, Kunio, Fuchu-shi, Tokyo 183-0012 (JP); FUKUDA, Kiyoshi, Ono-shi, Hyogo 675-1363 (JP); NAMBA, Kensuke, Ono-shi, Hyogo 675-1363 (JP); SHIMOAKA, Kaori, Ono-shi, Hyogo 675-1363 (JP)
(74) Representative: Müller-Boré & Partner Patentanwälte PartG mbB
(86) International application number: PCT/JP2019/025177
(87) International publication number: WO 2020/004393

(57) **Abstract**

The present invention relates to a pre-processing method for the purpose of formulating a drug substance having non-uniform particle sizes by a manufacturing method having excellent manufacture performance. According to the pre-processing method of the present invention, additives including at least a dispersant are blended in a drug substance having a specific particle size distribution and then the resultant mixture is deagglomerated/sized to disperse and make adhere the additives onto the surfaces of the particles of the drug substance, thereby yielding a powder having a specified particle size distribution. In this manner, a pharmaceutical preparation can be manufactured with excellent manufacture performance, manufacture efficiency, and manufacture cost by a direct compression method, a wet continuous granulation system or the like. Therefore, the pre-processing method is very useful.

## Description

### [Technical Field]

The present invention relates to a pre-processing method of a compression step, a granulation step or the like, in the manufacture of a pharmaceutical preparation using a drug substance having non-uniform powder physical properties (e.g., particle diameter, particle shape, density).

### [Background Art]

In the manufacture of a pharmaceutical preparation, as the method for manufacturing tablets among solid preparations, there are known some methods, such as a dry direct compression method (also simply referred to as a "direct compression method", hereinafter) in which a mixture of a drug substance and additives is compressed into tablets without preparing granules, and a method in which granules are prepared by any one of some methods such as a dry granulation method (e.g., a dry roller compacter method, a roll granulator method) or a wet granulation method (e.g., an agitation granulation method, an extrusion granulation method, a spray granulation method, fluidized-bed granulation method) and then the granules are compressed into tablets. In any one of these methods aside from just a few exceptions, scaling-up is required in the sifting of the manufacture to a commercial manufacture scale. A medicinal drug constitutionally has the highest priority to safety and efficaciousness, and the safety and efficaciousness are established in a transition development stage between a small-scale test manufacture step and an intermediate-scale test manufacture step or between an intermediate-scale test manufacture step and a commercial-scale manufacture step. In a product that is manufactured in a large quantity on a commercial manufacture scale, it is required that the quality of the product before scaling-up is in the same level as that after the scaling-up. However, it has been pointed out that a technique for quality control or the like still has some problems.

In the manufacture of a medicinal drug, a batch-wise manufacture has been common so far, and the introduction of continuous manufacture is delayed. However, the recent trend in the United States is that the development of continuous granulation systems has been demanded by the Food and Drug Administration (FDA) of the United States or the like, a guidelines or the like for the continuous granulation systems have been represented, and medicinal drugs manufactured by employing continuous manufacture have been approved. In Japan, the introduction of continuous manufacture has also been demanded by the Pharmaceuticals and Medical Devices Agency (PMDA). Furthermore, patent documents relating to continuous granulation devices and continuous granule manufacture systems have been published (e.g., Japanese Patent Laid-Open No. 2015-85225). One of the advantages of the continuous manufacture is that scaling-up is not needed or is easy to perform. In these situations, a continuous granulation system has been developed, in which a medicinal solid preparation having the same quality can be manufactured in a continuous manufacture system where a drug substance and additive powders are fed continuously in metered amounts during the manufacture process and a series of process including mixing 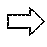 additive of a binder 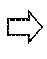 granulation 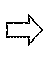 sizing 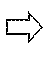 compression is carried out. For example, additives are blended in a drug substance, then the resultant mixture is fed to a continuous granulation system through a metered-dose feeder, and then a binder solution is added to the mixture. In this type of system, a method is recommended, in which the condition or state of the process or a product is monitored and controlled at each check point using a process analysis system (e.g., a PAT system) or the like so as to facilitate the scaling-up of a granulated product having the same quality. However, in the scaling-up in, for example, batch-wise fluidized bed granulation, there is a problem that the amounts (kg/m²: loading density) of components to be charged per unit area increase and the loaded thickness also increases with the increase in the size of the device and, therefore, the pressure against particles during granulation also increases. Under these circumstances, for the manufacture of a preparation having the same quality before and after the scaling-up, there are still various problems.

It is well-known that the powder physical properties (e.g., particle diameter, shape, particle density) of particles of a drug substance to be processed vary depending on the manufacturers of the drug substance, and also vary depending on the seasons (summer and winter) in which the drug substance is manufactured and the lot numbers of the drug substance even in the same manufacturer. If the powder physical property of particles of a drug substance varies, the quality of the product in the latter step also varies. Therefore, in this case, it is difficult to yield a product of which the quality is maintained at the same level. However, a pre-processing method for uniformizing particles of a drug substance for overcoming the problem of variations in quality associated with the change in season or the like is unknown.

The particle diameters of a drug substance or an additive which has been used before around 1970 were relatively large, and there were many drug substances having high solubility. Accordingly, even in a drug substance or an additive having non-uniform particle sizes, there was no problem about solubility and dissolution rate. Furthermore, since an air conditioning facility used in a working environment for the manufacture of a medicinal drug at that time was in bad condition and therefore the humidity in the working environment was high (left it be), there was very few harmful effect of the secondary aggregation of the drug substance or the additive or the like and a main force applied to particles of the drug substance or additive was gravity (mass).

In contrast, many of the recent drug substances are hardly soluble. In many case, a drug substance is finely pulverized using a pin mill, a hammer mill, a jet mill or the like in a manufacturer of the drug substance for the purpose of improving the solubility or the like of the drug substance, Therefore, drug substance manufacturers have supplied (sold on the market) drug substances having non-uniform powder physical properties. In association with the fine pulverization of a drug substance, the charging with static electricity, an intermolecular force (van der Waals force), a surface energy or the like has a great effect. In the case of a drug substance having extremely high solubility, even when an insoluble additive is blended, the highly soluble drug substance selectively binds to and coagulates with mists of a binder precedently upon the addition of a solution of the binder with a spray. As a result, a gel (undissolved lumps of powder) may be formed on the surfaces of the drug substance powder, causing the delay of the dissolution of the drug substance. Particularly in the preparation of a drug substance added in a small amount and having a specific color hue, a problem may occur with respect to uniformity such as unevenness in color or formation of dots in the final product.

The powder physical properties (e.g., particle size distribution, particle shape, density) in the synthesis of a drug substance also vary depending on the types of the environment in which a facility is placed in each drug substance manufacturer. It is well-known that, even in the same facility in the same manufacturer, the variations in season (e.g., the difference in temperature or humidity between summer and winter) in which the drug substance is manufactured, the environment in which a facility is placed or the like has large influence. For example, in many cases, the environment in which a plant for the synthesis/crystallization process for a drug substance is placed is almost outdoor rather than an environment which is fully air-conditions and in which the temperature is carefully controlled as in the case of the preparation manufacture process or the like. Particularly the control of temperature in a cooling step that is the final step in the synthesis of a drug substance is almost left it be, and there are very few cases where the drug substance is manufactured in a fully air-conditioned environment. Accordingly, the cooling rate in the cooling step varies greatly depending on the size of the device and the type of the environment in which the facility is placed. As a result, the powder physical properties (e.g., particle diameter, particle size distribution, particle shape, particle density, electrical chargeability, intermolecular force) of the drug substance manufactured inevitably vary.

For the above-mentioned reasons, the synthesized drug substance contains large-size crystals formed in the crystallization step in the process of the manufacture, masses generated as the result of secondary aggregation or the like, and often contains large particles each having a particle size of 500 µm or more in a large amount. Therefore, the synthesized drug substance has non-uniform particle sizes. Particularly when the particle sizes are non-uniform in a hardly soluble drug substance and the variations in seasons are large, the uniformly in the dissolution rate of the final product (e.g., tablets) cannot be expected. In order to overcome these problems, drug substance manufacturers have supplied (sold on the market) drug substances each in the form finely pulverized with a jet mill, a pin mill, a hammer mill or the like. However, since fine particles each having a size of 50 to 60 pm or less are likely to be excessively pulverized to cause secondary aggregation, medicine (pharmaceutical) manufacturers producing fine granules, granules, capsules, tablets or the like have had difficulty with the manufacture of pharmaceutical preparation. For example, it is extremely difficult to disperse secondary-aggregated small masses even using a shaking mixer. The small masses are sometimes removed using a shaking sieve. However, in this case, the charging with static electricity is further promoted by the shaking, resulting in the acceleration of secondary aggregation. Therefore, the removal of the small masses with a shaking sieve is improper. For these reasons, in some cases, the mixing has been carried out after a trituration step (i.e., a simple mixing step). However, a step of deagglomerating/sizing and dispersing secondary-aggregated drug substance particles as in the present application is not carried out in the past. As mentioned above, even when additives are mixed with a drug substance of which the powder physical properties vary depending on seasons and the resultant mixed powder is introduced into a tablet pressing machine or a wet granulation system, the physical properties (e.g., particle diameter, shape, density) of fine granules, granules, capsules or tablets obtained may varied, and it is difficult to manufacture a final product having the same quality in every step.

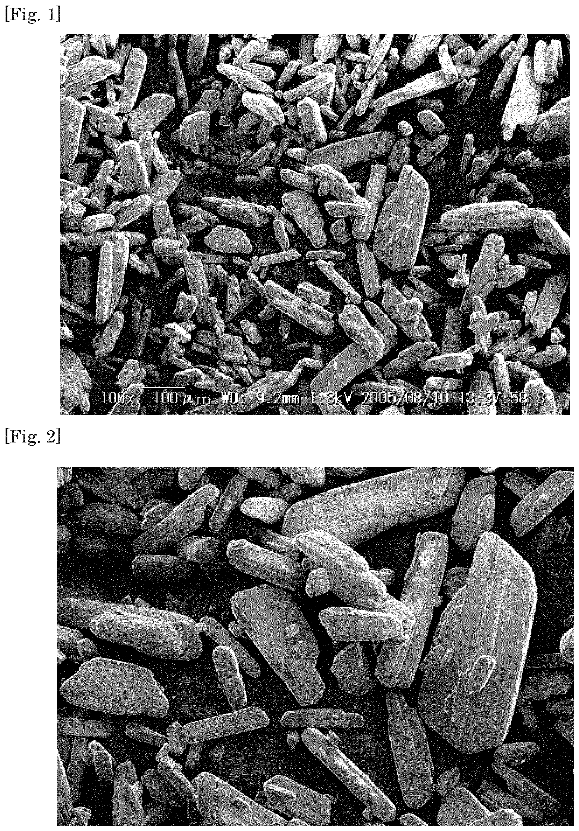

Drug substance fine particles each having a particle size of 50 to 60 µm or less can be charged with static electricity extremely strongly, and therefore exist in the form of secondary-aggregated particles. A large non-uniform particle having a particle size of 500 µm or more seems like a single particle, but is actually a particle composed of needle crystals, flaky crystals or the like accumulated or layered on each other (see photograph 1 (Fig. 1) and photograph 2 (Fig. 2; a center-enlarged view of photograph 1)). When the particle is pulverized by strong impact power, the particle is broken into fine particles each having a particle size of 50 to 60 µm or less, and secondary-aggregated particles are formed.

As a harmful effect of the excessive pulverization of a drug, it is well-known that, in the case of ibuprofen that has a melting point of 74°C to 77°C, when ibuprofen is pulverized by strong impact power using a pin mill, a hammer mill or the like, heat is generated and ibuprofen is firmly adhered to a pulverization pin or an inner wall due to the heat, which makes a continuous operation difficult. In the case of a commercially available acetaminophen drug substance, particles pulverized by strong impact power generated by a pin mill, a hammer mill or the like are supplied (sold on the market). Therefore, many secondary-aggregated particles are contained and many needle particles are also contained, resulting in the deterioration in flowability. Therefore, even when other additive is blended in the drug substance and the resulting mixture is fluidized-bed-granulated in the batch-wise fluidized bed granulation, the deterioration in flowability or a pass-through phenomenon occurs and therefore the achievement of a smooth fluid state cannot be expected. In spite of these situations, there are still many cases where the operation is carried out in an excessive air flow amount for the purpose of forcibly causing flowing. In these cases, however, the excessive air flow amount at an early stage of the flowing makes the drug substance particles having micropowdery form float and fluidize precedently and, as a result, the drug substance particles are adhered onto an antiscattering bag filter that is installed in an upper part. Even when drug substance particles having strong chargeability with static electricity are subjected to a dust removal operation such as shaking, it is extremely difficult to circulate the drug substance particles in a spray zone, which may cause the deterioration in content uniformity. Moreover, even when the mixture are shaked/agitated using a batch-type agitation granulator (e.g., a vertical granulator; Powrex Corporation), the resultant particles are uniform as a whole, but there are found secondary-aggregated small masses locally. Since the dispersion of the secondary-aggregated small masses is extremely difficult, the granulation proceeds while keeping the forms of the secondary-aggregated small masses, sometimes resulting in the problem in content uniformity such as the unevenness in color, the formation of dots or the like.

### [Summary of the Invention]

### [Problem to be Solved by the Invention]

The present invention provides a pre-processing method for preparing a drug substance having non-uniform powder physical properties, particularly non-uniform particle sizes. Particularly, the problem to be achieved by the pre-processing method according to the present invention (also referred to as the "present pre-processing method", hereinafter) is to provide a pre-processing method for a tablet compression step or a granulation step in the manufacture of a pharmaceutical preparation, whereby it becomes possible to manufacture a powder that is highly applicable to a manufacture method employing a dry direct compression method or a continuous granulation system for which excellent flowability and uniformity of a powder are required more than other manufacture methods. The present pre-processing method is a sizing method, and therefore is not always carried out prior to a tablet compression step or a granulation step.

Recent drug substances each of which has a high physiological activity, is hardly soluble and has high aggregability are finely pulverized using a jet mill, a pin mill, a hammer mill or the like in order to improve the solubility of the drug substances. However, when fine particles each having a particle size of 50 to 60 pm or less are pulverized with a pin mill, a hammer mill or the like, the fine particles are often pulverized excessively. As a result, the charging with static electricity, an intermolecular force (van der Waals force), a surface energy or the like affects strongly to cause the secondary aggregation of the powder, resulting in the manufacture of a drug substance having extremely low flowability. Therefore, in order to improve the flowability of the drug substance to improve mixing uniformity in the process of preparation, a trituration step (i.e., a simple mixing step) is carried out in advance, and then the mixture is mixed with other additives. In some cases, the trituration step is carried out in one stage, two stages, or three stages. However, the manufacture efficiency may be deteriorated with the increase in the number of manufacture steps. There are also cases where it is attempted to improve the flowability or the like of a drug substance by blending a large quantity of additives in a granulation operation or the like. However, the dispersion of secondly-aggregated drug substance fine particles is difficult. Therefore, there is yet found no excellent method that can deal with these problems. Furthermore, the dispersion of the secondary-aggregated drug substance particles or the like in a binder solution is likely to become non-uniform. As a result, the drug substance particles may be partially secondary-aggregated which results in the deterioration in a dissolution rate, or the drug substance particles may become in the form of granules containing small masses which results in the slight deterioration in content uniformity. Furthermore, the finely pulverization step may cause the deterioration in yield or the increase in cost due to the adhesion of the particles onto a wall surface of the device or the like. Furthermore, some drugs have the problem that the drugs are unstabilized by to a strong mechanical force such as pulverization or the generation of a pressure, an impact, heat or the like generated during the compression molding/compression into tables. Then, the present inventors have studied in order to address the problem of providing a pre-processing method to be employed in the manufacture of a pharmaceutical preparation, whereby the flowability of a drug can be improved, the solubility and uniformity of the drug can be improved by controlling the particle sizes of particles by an action to grinding only larger particles selectively without carrying out excessive pulverization, and the manufacture performance of the drug can also be improved.

### [Means for Solving the Problems]

The present inventors have made extensive and intensive studies for solving the above-mentioned problems. As a result, it is found that: in the preparation of a drug, when a drug substance of which the powder physical properties (e.g., particle diameter, shape, particle density) vary among the drug substance manufacturers as well as the seasons (e.g., summer and winter) or the lot numbers manufactured in the same drug substance manufacturer and which therefore has non-uniform particle sizes is used, it is important to blend additives including a dispersant in the drug substance and deagglomerate/size the resultant mixture once or a plurality of times to disperse and make adhere the additives including the dispersant in/onto the surfaces of the particles of the drug substance and to impart a specified particle size distribution to the powder. In this regard, the additives including a dispersant to be added to the drug substance may be any one as long as the flowability can be improved and the occurrence of secondary aggregation can be prevented, and it is not necessary required to form a continuous layer. For example, a dispersant and optionally other additive are added to a drug substance which contains particles each having a particle size of 500 pm or more in an amount of 1% by weight or more and particles each having a particle size of 60 pm or less in an amount of 10% by weight or more both relative to 100% by weight of the drug substance when the particle size distribution is measured by a sieving method, and then the resultant mixture is deagglomerated/sized to disperse and make adhere the additives including the dispersant in/onto the surfaces of the particles of the drug substance. In this manner, a powder is prepared, which contains particles each having a particle size of 180 pm or more in an amount of 25% by weight or less and particles each having a particle size of 60 pm or less in an amount of 25% by weight or less both relative to 100% by weight of the powder when the particle size distribution is measured by a sieving method . Alternatively, a dispersant and optionally other additive are added to a drug substance which contains particles each having a particle size of 500 pm or more in an amount of 1% by volume or more and particles each having a particle size of 50 pm or less in an amount of 10% by volume or more both relating to 100% by volume of the drug substance when the particle size distribution is measured by a laser-diffraction method, and then the resultant mixture is deagglomerated/sized to disperse and make adhere the additives including the dispersant in/onto the surfaces of the particles of the drug substance. In this manner, a powder is prepared, which contains particles each having a particle size of 200 pm or more in an amount of 50% by volume or less and particles each having a particle size of 50 pm or less in an amount of 70% by volume or less both relative to 100% by volume of the powder when the particle size distribution is measured by a laser-diffraction method. In this manner, it is found that the flowability, solubility and uniformity can be improved and the range of variations in powder physical properties (e.g., particle diameter, particle size distribution, particle shape, density) can be reduced without the need to use a plurality of additives in larger amounts.

In general, for the improvement in solubility, importance has been placed on increasing a specific surface area. However, it is found that the modification of the surfaces of particles of a drug substance is also important and that, even if the specific surface area is unchanged, the dissolution rate can be improved by making a solubilizing agent or a polymer binder adhere and layer onto the surfaces of the particles of the drug substance.

On the basis of these findings, by employing the present pre-processing method in the process of the manufacture of a pharmaceutical preparation, it becomes possible to manufacture tablets by a dry direct compression method (the simplest method), which has been believed to be difficult when a drug substance having non-uniform particle sizes and low flowability is used. Furthermore, by feeding a powder prepared by the present pre-processing method to a continuous granulation system, it becomes possible to manufacture granules having excellent uniformity, and it also becomes possible to manufacture a solid pharmaceutical preparation having stable quality. In these cases, granules or tablets having excellent content uniformity and therefore having excellent product quality can be manufactured in a batch-wise fluidized bed granulation method or a batch-wise agitation granulation method.

For large crystal (e.g., needle or columnar crystals) contained in the drug substance, in order to make the crystals into particles having a (longer diameter)/(shorter diameter) ratio of 3 or less, desirably spherical particles, it is important to deagglomerate the crystals selectively by using a device which can deagglomerate, size and disperse particles by an action to grind particles with small impact power and without rarely generating heat, such as a grinding stone-type mill [e.g., trade name: Supermasscolloider (registered trademark, Masuko Sangyo Co., Ltd.)] and a rod-shaped or impeller-type deagglomerating/sizing machine [trade name: Comil (registered trademark, Powrex Corporation)]. It is found that a preparation, e.g., small-size tablets and sustained-release tablets, which is improved in aggregability and low flowability of the drug, has excellent dissolution properties and shapability and is bitterness-masked can be manufactured by selectively deagglomerating/sizing crystals of the drug substance which have large particle diameters or aggregated mass of the drug substance using a deagglomerating/sizing machine to disperse and make adhere the additives including the dispersant in/onto the surfaces of the particles of the drug substance at a stage where a dispersant and optionally a solubilizing agent are blended in the drug substance or a stage where other additive is further added.

The term "pulverization" and the term "deagglomeration/sizing/dispersion" as used herein are defined as follows. (1) The term "pulverization" refers to a unit operation for applying strong impact on larger particles using a hammer mill, a pin mill, a jet mill or the like to reduce the size of the particles, while (2) the term "deagglomeration/sizing/dispersion" refers to a unit operation for applying slightly weak impact on particles of a drug substance which are aggregated particles or (e.g., needle, columnar or flaky) crystals accumulated or stacked on each other to deagglomerate/disperse/size the particles. The operation of the deagglomeration/sizing/dispersion is similar to that of the pulverization and partially overlaps with the pulverization. These terms are clearly distinguished from each other in the art of powder industry, although these terms are sometimes confused with each other.

On the other hand, when the amount of the additive is reduced for reducing the size of tablets, the shapability of the tablets may be deteriorated and the hardness of the tablets may become insufficient. However, it is found that tablets having excellent hardness can be manufactured by adding a small amount of water in the process of the manufacture to adjust the water content in the powder and then pressing the powder into tablets. The present inventors have accomplished the present invention on the basis of these findings.

The present invention relates to, for example, the following items (1) to (13), but is not limited to these items.
(1) A pre-processing method in the manufacture of a pharmaceutical preparation, comprising: adding a dispersant and optionally other additive to a drug substance which contains particles each having a particle size of 500 pm or more in an amount of 1% by weight or more and particles each having a particle size of 60 pm or less in an amount of 10% by weight or more relative to 100% by weight of the drug substance; and then carrying out deagglomeration/sizing of the mixture to disperse and make adhere at least the dispersant and the other additive in/onto the surfaces of particles of the drug substance, thereby manufacturing a powder that contains particles each having a particle size of 180 pm or more in an amount of 25% by weight or less and particles each having a particle size of 60 pm or less in an amount of 25% by weight or less relative to 100% by weight of the powder, wherein the particle size distribution of the drug substance and the powder is measured by a sieving method.
(2) A pre-processing method in the manufacture of a pharmaceutical preparation, comprising: adding a dispersant and optionally other additive to a drug substance which contains particles each having a particle size of 500 µm or more in an amount of 1% by volume or more and particles each having a particle size of 50 pm or less in an amount of 10% by volume or more relative to 100% by volume of the drug substance; and then carrying out deagglomeration/sizing of the mixture to disperse and make adhere at least the dispersant and the other additive in/onto the surfaces of particles of the drug substance, thereby manufacturing a powder that contains particles each having a particle size of 200 pm or more in an amount of 50% by volume or less and particles each having a particle size of 50 pm or less in an amount of 70% by volume or less relative to 100% by volume of the powder, wherein the particle size distribution of the drug substance and the powder is measured by a laser-diffraction method.
(3) The method according to item (1) or (2), wherein at least one of microcrystalline cellulose in an amount of 0 to 85% by weight, a disintegrating agent in an amount of 0 to 30% by weight, a surfactant (solubilizing agent) in an amount of 0 to 6% by weight, a water-soluble additive in an amount of 0 to 40% by weight and a sugar alcohol in an amount of 0 to 15% by weight each relative to 100% by weight of the preparation is blended and the resultant mixture is deagglomerated/sized at least one time to disperse and make adhere the additive in/onto the surfaces of the particles of the drug substance.
(4) The method according to any one of items (1) to (3), wherein water in an amount of 0.5 to 3.0% by weight relative to 100% by weight of the preparation is added if necessary.
(5) The method according to any one of items (1) to (4), wherein the pre-processing method is for a dry direct compression method.
(6) The method according to any one of items (1) to (4), wherein the pre-processing method is for a granulation step.
(7) The method according to item (6), wherein the granulation step is carried out in a continuous granulation system.
(8) The method according to any one of items (1) to (7), wherein the drug substance is low flowable, hardly soluble, or highly soluble but capable of forming a gel (undissolved lumps of powder).
(9) The method according to any one of items (1) to (8), wherein the drug substance is pregabalin, celecoxib, acetaminophen or ibuprofen.
(10) The method according to any one of items (1) to (9), wherein the dispersant is hydrated silicon dioxide, light anhydrous silicic acid or calcium silicate.
(11) The method according to any one of items (1) to (10), wherein the additive other than the dispersant is at least one component selected from: an aminoalkyl methacrylate copolymer E, an aminoalkyl methacrylate copolymer L, an aminoalkyl methacrylate copolymer LD, a methacrylic acid copolymer S, an ammonioalkyl methacrylate copolymer, microcrystalline cellulose, low-substituted hydroxypropylcellulose, crospovidone, light anhydrous silicic acid, hydrated silicon dioxide, calcium silicate, carboxymethyl starch sodium, titanium oxide, iron oxide, talc, starch, a lubricant; a water-soluble additive selected from a carboxyvinyl polymer, hydroxypropyl cellulose, polyvinylpyrrolidone, a polyvinyl alcohol-acrylic acid-methyl methacrylate copolymer, polyvinyl alcohol, a polyvinyl alcohol-polyethylene glycol-graft copolymer, copolyvidone, hydroxypropyl methylcellulose, lactose, a saccharide, a sugar alcohol and trehalose; and a surfactant (solubilizing agent) selected from macrogol, sodium lauryl sulfate and polysorbate.
(12) The method according to any one of items (1) to (11), wherein the deagglomeration/sizing is carried out using a grinding stone-type mill and/or a rod-shaped or impeller-type deagglomerating/sizing machine.
(13) The method according to any one of items (1) to (12), wherein each of the deagglomeration/sizing using a grinding stone-type mill and the deagglomeration/sizing using a rod-shaped or impeller-type deagglomerating/sizing machine is carried out at least one time.

### [Advantages of the Invention]

According to the present pre-processing method, it becomes possible to prepare a powder containing a drug in a broad blending ratio (0.5 to 98% by weight). When the powder is used, the drug content uniformity is excellent, the scaling-up from a test manufacture scale to a commercial manufacture scale can be achieved easily, and the manufacture process can be simplified, resulting in the reduction in manufacture cost. Furthermore, according to the present pre-processing method, since flowability and solubility can be improved, it is not needed to use many types additives in large amounts and therefore it becomes possible to manufacture tablets having smaller sizes compared with the conventional tablets. The powder prepared by the present pre-processing method also has an auxiliary effect that, even in the case of a drug substance which is likely to undergo tablet pressing failures upon the direct contact with the metal surface of a mortar or/and a pestle in a tablet pressing machine, the surfaces of particles of the drug substance are coated with particles of an additive such as a dispersant, an insoluble additive, a surfactant (solubilizing agent) and a water-soluble polymer and therefore the occurrence of tablet pressing failures can be reduced. Furthermore, when the powder prepared by the present pre-processing method is subjected to a dry direct compression method, the occurrence of the secondary aggregation of the powder can be reduced and high tablet hardness can be secured by adjusting the water content to a value suitable for tablet compression (wherein the amount of water to be added for the adjustment of the water content may vary depending on the physical properties and blending ratios of a drug substance or an additive, and is generally 0.5 to 3.0% by weight relative to the whole amount, i.e., 100% by weight, of the powder).

### [Brief Description of Drawings]

Fig. 1 is an SEM image (×100 magnification) of an ibuprofen drug substance powder having a lot number A (photograph 1).
Fig. 2 is a center-enlarged SEM image (×200 magnification) of photograph 1 (the ibuprofen drug substance powder A) (photograph 2).
Fig. 3 is an SEM image (×100 magnification) of an ibuprofen drug substance powder having a lot number B (wherein the lot is manufactured by the same manufacturer in a different season from the season in which the product having a lot number of A is manufactured) (photograph 3).
Fig. 4 is an SEM image (×100 magnification) of an ibuprofen deagglomerated/sized product (photograph 4).

### [Mode for Carrying Out the Invention]

The present invention relates to a pre-processing method, e.g., a tablet compression step or a granulation step, in the manufacture of a pharmaceutical preparation, the pre-processing method comprising blending additives including at least a dispersant to a drug substance (A) having a specific particle size distribution and then carrying out deagglomeration/sizing of the resultant mixture once or a plurality of times to disperse and make adhere the additives in/onto the surfaces of particles of the drug substance, thereby yielding a powder (B) having a specified particle size distribution. In the present invention, the particle size distribution of each of the drug substance (A) and the powder (B) can be determined by a weight-based distribution evaluation by a sieving method (measurement device: a ro-tap-type sieve shaker or the like) and a volume-based distribution evaluation by a laser-diffraction method (also referred to as "a laser diffraction scattering method") (measurement device: Mastersizer 2000 or Mastersizer 3000 [Malvern] or the like, dispersion compression air pressure: 2 to 4 bars). Based on the results measured in each of the methods, the range (upper limit/lower limit) of the particle diameter is set as mentioned in [1] or [2] below.
[1] In the case where a particle size distribution is measured by a sieving method, a drug substance (A) containing particles each having a particle size of 500 pm or more in an amount of 1% by weight or more and particles each having a particle size of 60 µm or less in an amount of 10% by weight or more, preferably containing particles each having a particle size of 500 pm or more in an amount of 10% by weight or more and particles each having a particle size of 60 pm or less in an amount of 13% by weight or more, each relative to 100% by weight of the drug substance (A); and a powder (B) containing particles each having a particle size of 180 µm or more in an amount of 25% by weight or less and particles each having a particle size of 60 pm or less in an amount of 25% by weight or less, preferably containing particles each having a particle size of 180 µm or more in an amount of 20% by weight or less and particles each having a particle size of 60 µm or less in an amount of 20% by weight or less, each relative to 100% by weight of the powder (B).
[2] In the case where a particle size distribution is measured by laser diffraction method, a drug substance (A) containing particles each having a particle size of 500 pm or more in an amount of 1% by volume or more and particles each having a particle size of 50 pm or less in an amount of 10% by volume or more, preferably containing particles each having a particle size of 500 pm or more in an amount of 3% by volume or more and particles each having a particle size of 50 pm or less in an amount of 15% by volume or more, each relative to 100% by volume of the drug substance (A); and a powder (B) containing particles each having a particle size of 200 µm or more in an amount of 50% by volume or less and particles each having a particle size of 50 pm or less in an amount of 70% by volume or less, preferably containing particles each having a particle size of 200 pm or more in an amount of 40% by volume or less and particles each having a particle size of 50 µm or less in an amount of 50% by volume or less, each relative to 100% by volume of the powder (B).

The powder prepared by the pre-processing method is improved in flowability or the like, and is reduced in fluctuations in powder physical properties and has high uniformity, and therefore has high manufacture performance. Accordingly, in the formulation into tablets or the like using the powder prepared by the pre-processing method, a dry direct compression method excluding a granulation step or a continuous granulation system in which quality control is difficult to achieve can apply, and the number of types or quantities of additive can be reduced, resulting in the achievement of the manufacture of tablets or the like which are small in size and contain an active ingredient at a high content or the reduction in the cost for the manufacture.

Furthermore, in the present pre-processing method, it is critical to manufacture a powder having the above-mentioned specified particle size distribution by deagglomerating/sizing a powder prepared by mixing a drug substance having the above-mentioned specified particle size distribution with the additives using a deagglomerating/sizing machine to disperse and make adhere the additives including a dispersant, a solubilizing agent and the like in/onto the surfaces of particles of the drug substance. In the present pre-processing method, the device to be used for the deagglomeration/sizing is not particularly limited, and a deagglomerating/sizing machine is suitable which can control the particle size by action to grind larger particles selectively with a grinding stone (grinder) or a rod-shaped or impeller-type rotary body (number of rotations: about 800 to about 3000 rpm). For example, a grinding stone-type mill (Supermasscolloider [registered trademark]) can be mentioned, which has a function that an introduced raw material powder is fed to a gap between two poreless grinders which are arranged one above the other and of which the spacing can vary freely, and is then ground in the gap by the action of compression, shearing, rolling friction or the like generated in the gap and is therefore gradually rounded and becomes more smooth. In the present pre-processing method, the clearance (i.e., the gap between grinding stones) of the grinding stone-type mill is generally 30 to 5000 µm, preferably 500 to 3000 µm, more preferably 1000 to 2000 µm. In addition, a rod-shaped or impeller-type deagglomerating/sizing machine (e.g., Comil) can also be mentioned, which has a function that an introduced raw material powder is deagglomerated/sized by pressing the raw material power against a cylinder-shaped screen by the action of a centrifugal force caused by a rotating impeller (rotating vane) and then the raw material powder is discharged through a plurality of openings provided in the screen. The screen diameter (i.e., the diameter of each of the openings in the screen) in the deagglomerating/sizing machine is preferably about 0.5 to 4 mm.

On the other hand, as a processing for making particles fine like "deagglomeration", "pulverization" can be mentioned. A pulverizing machine is a device in which a hammer or a pin rotates at a high speed (the number of rotations: about 5000 to 15000 rpm) to apply strong impact, e.g., compression, impact, friction and shearing, onto raw material powder particles to make the particles fine, and can process particles particularly regardless of the sizes of the particles. Accordingly, in the case of acetaminophen for example, when a powder of acetaminophen is finely pulverized using a pulverizing machine such as a pin mill, a hammer mill and a jet mill, small particles are pulverized excessively and, as a result, specific surface areas are increased and the influence of charged static electricity or an intermolecular force increases. As a result, the flowability of the particles decreases and the secondary aggregation of the particles occurs, which may cause the adhesion of the particles onto the inner wall surface of the device or the like, leading to the deterioration in workability.

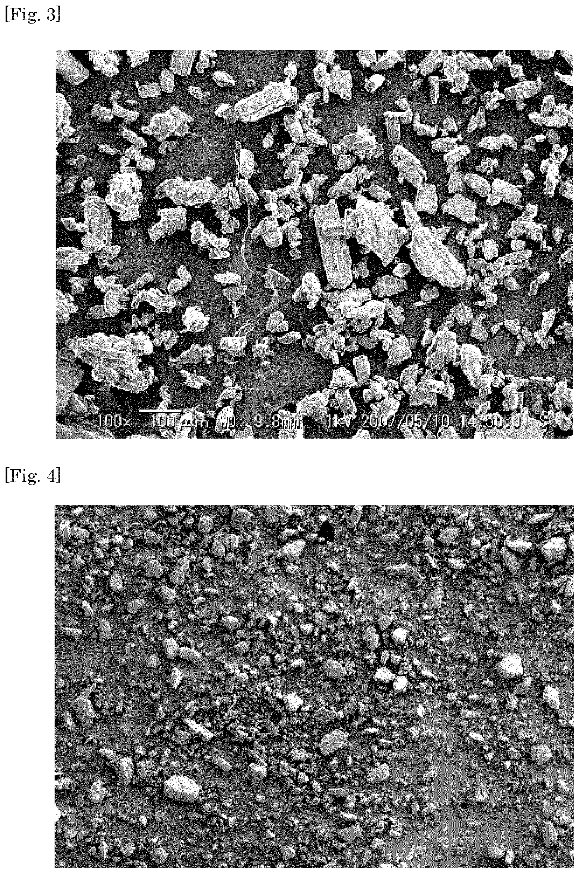

In Figs. 1 and 2, electron microscopic images of particles of an ibuprofen drug substance before deagglomeration/sizing are shown (photograph 1: ×100 magnification, photograph 2: a center enlarged view, ×200 magnification). In Fig. 4, an electron microscopic image of the particles of the ibuprofen drug substance after deagglomeration/sizing is shown (photograph 4: ×100 magnification). In photograph 1, each of the particles of the ibuprofen drug substance appears like a large single particle. However, in photograph 2 which is a center-enlarged view of photograph 1, it is confirmed that crystals are stacked and accumulated; while in photograph 4, it is confirmed that stacked and accumulated particles are deagglomerated and dispersed. When comparison is made between photograph 1 and photograph 3, it is confirmed that, even in powders produced by the same manufacture, the particle sizes, particle shapes and the like vary depending on the seasons (summer and winter) in which the powders are manufactured.

The melting point of ibuprofen is about 74 to about 77°C. It is known that, when the particles of the ibuprofen drug substance are pulverized with a pin mill, a hammer mill or the like, the particles are firmly adhered onto an inner wall surface of the device, a pulverization pin, a hammer or the like by the action of heat generated upon impacting and therefore long-term continuous pulverization (operation) is difficult. Even in the case of a drug substance having a low melting point and having some difficulties in handling (e.g., manufacture easiness) like ibuprofen, the number of slightly round-shaped particles increases while suppressing the generation of heat and therefore the flowability of the particles can be improved by carrying out deagglomeration/sizing of the particles. Some drugs such as pregabalin and candesartan may become unstable due to strong mechanical impact such as pulverization or generation of heat. Therefore, the present pre-processing method, in which the particle sizes of larger particles can be controlled selectively while preventing the occurrence of impaction or the generation of heat by a deagglomerating/sizing machine and avoiding excessive pulverization, can reduce the unstabilization of the drugs and is therefore useful.

For the above-mentioned reasons, in the present pre-processing method, it is important that coarse crystals or masses of a drug are deagglomerated or made fine selectively, particles in a fine powder zone are dispersed uniformly without carrying out excessive pulverization, and an additive such as a dispersant is made adhere onto the surfaces of the particles uniformly, in other words, a powder having a specified particle size distribution is manufactured by deagglomeration/sizing, without employing the conventional pulverization method. Particularly in the case of long and thin needle crystals of a drug among crystals of drugs, the crystals can be dispersed more uniformly by adjusting the particle diameters of the crystals by the deagglomeration/sizing of the crystals in such a manner that the (longer diameter)/(shorter diameter) ratio can become 3 or less. In this manner, the charged static electricity or the intermolecular force in the drug can be reduced. As a result, the flowability and coagulation properties of the drug can be improved, resulting in the further improvement in manufacture performance. Particularly in the case where a drug which has non-uniform particle diameters, such as a drug which contains, in the process of the manufacture of the drug, larger crystals generated during a crystallization step, masses of the crystals due to secondary aggregation or the like, is used, for the purpose of avoiding the further overpulverization of drug particles having smaller particle diameter to cause secondary aggregation of the particles, it is preferred to select a deagglomeration/sizing processing that enables drug particles having larger particle diameters to be finely divided selectively so as to control the particle diameters of the particles. In the present pre-processing method, it is also preferred to carry out the deagglomeration/sizing step not only one time but also desired times depending on the blending ratios and blending amounts of the drug substance and additives, the types of the operation environments and the like. In the case of a powder prepared by blending additive in the drug substance, the particle diameters of the additives (e.g., microcrystalline cellulose, a disintegrating agent) become dominant. Therefore, the particle diameters of the powder are often expressed in larger values than the particle diameters of the drug substance.

In recent years, in drug substance manufactures, there are many cases where crystals generated in a crystallization step in the synthesis of a drug substance are finely pulverized to an average particle diameter of 50 to 60 µm or less. In these cases, a hardly soluble drug substance has a strong secondary aggregation force, and therefore may have difficulty in flowability and uniformity or may be decreased in yield due to the adhesion onto the inner wall surface of a pulverization device. However, when a drug substance is mixed with a dispersant (e.g., Carplex, Aerosil) and optionally other additives such as a surfactant (solubilizing agent) and larger particles in the resultant mixture are selectively deagglomerated/sized and are uniformly dispersed using a grinding stone-type mill or an impeller-type deagglomerating/sizing machine each having weak impact power, the occurrence of re-coagulation of particles can be prevented and the flowability and dissolution properties of the particles can be improved.

The present pre-processing method can prepare a powder which can be used in a dry direct compression method or can be fed to a wet granulation system, and can prepare a powder having a specified particle size distribution by blending a dispersant (e.g., hydrated silicon dioxide (Carplex), light anhydrous silicic acid (Aerosil)) in an amount of 0.1 to 6.0% by weight in a drug substance having the above-mentioned particle size distribution, then carrying out deagglomeration/sizing of the resultant mixture to disperse these components, then adding other additives (e.g., a water-soluble polymer binder, a sugar alcohol, trehalose, a carboxyvinyl polymer) to the powder, and then carrying out deagglomeration/sizing of the resultant mixture to disperse and make adhere the additives in/onto the surfaces of particles of the drug substance. By using the present powder, even when the drug substance is low flowable and highly electrically chargeable and is therefore likely to cause disadvantages in a dry direct compression method, it becomes possible to manufacture tablets having excellent content uniformity.

Furthermore, in the present pre-processing method, (1) a low flowable drug substance and a dispersant (e.g., hydrated silicon dioxide, light anhydrous silicic acid) are deagglomerated/sized once or a plurality of times to disperse these components uniformly, (2), if necessary, the water content in the resultant mixture is adjusted to a value suitable for the compression into tablets, (3) an insoluble additive (e.g., microcrystalline cellulose, a disintegrating agent) is further blended in the mixture, and (4) a water-soluble polymer binder (e.g., a carboxyvinyl polymer, hydroxypropyl cellulose) is dispersed/made adhere (coat) in/onto the surfaces of particles of the low flowable drug substance by carrying out deagglomeration/sizing once or a plurality of times. In this manner, a premix drug substance which has improved electrical chargeability and flowability and rarely undergoes secondary aggregation can be manufactured. In the case where a plurality types of drug substances are blended, when the drug substances are deteriorated upon the contact with each other, an intermediate layer may be provided in order to avoid the contact of the drug substances with each other.

Due to the increase in stringentness of shipping standards of drug substances and additives in drug substance manufacturers and additive manufacturers, many of the products of drug substances and additives are provided in an excessively dried form having a water content value smaller than a value (0.5 to 3.0% by weight) suitable for the compression into tables. As a result, a drug substance is increased in charged static electricity, an intermolecular force (van der Waals force) and a surface energy and is likely to undergo secondary aggregation. Therefore, in the formulation of the drug, a problem about content uniformity may occur. Furthermore, the hardness of the tablets may be decreased. In the present pre-processing method of the present invention, the water content is adjusted to a value suitable for the compression into tables (i.e., water is added in an amount of 0.5 to 3.0% by weight relative to the whole amount, i.e., 100% by weight, of the powder) if necessary and, as a result, the charged static electricity, the intermolecular force (van der Waals force) and the surface energy in the drug substance can be reduced. In this manner, the problem about the content uniformity caused by secondary aggregation and the problem about the hardness of tablets in the formulation of the drug substance can be overcome.

In the case of a hardly soluble drug substance, the surface modification with a surfactant (solubilizing agent) is sometimes effective for the improvement in the solubility of the drug substance. Therefore, it is possible to add a powdery surfactant (solubilizing agent) such as powdery macrogol and sodium lauryl sulfate as it is or in the form dissolved in water for water content control purpose, and then carrying out deagglomeration/sizing once or a plurality of times to disperse and make adhere the surfactant uniformly in/onto the surfaces of the particles of the drug substance.

A kind of the drug that can be used in the present pre-processing method is not particularly limited as long as the drug can be used as a medicinal drug for the prevention or treatment of diseases. A single drug may be used or a plurality of drugs may be used in a blended form. Examples of the drug include pregabalin, duloxetine, celecoxib, candesartan, valsartan, olmesartan medoxomil, amlodipine besilate, phenytoin, nizatidine, bucillamine, azelnidipine, loxoprofen sodium hydrate, acetaminophen, ibuprofen, noscapine, caffeine, famotidine, levofloxacin hydrate, ethenzamide and tramadol hydrochloride. Each of these drugs has a large amount of charged static electricity, a large intermolecular force and a large surface energy, and therefore has easily secondary-aggregatable powder physical properties. Therefore, these drugs are suitable for achieving the effects of the present pre-processing method. In the present invention, the blending ratio of the drug is not particularly limited. The drug can be contained in an amount falling within a wide range, i.e., 0.5 to 98% by weight, relative to 100% by weight of the final preparation.

In the case where tablets are manufactured by carrying out the present pre-processing method, even when a dry direct compression method excluding a granulation step is employed, bindability and sustained release performance can be achieved by uniformly dispersing (coating)/adhering an insoluble additive and/or a water-soluble polymer additive in/onto the surfaces of the particles of the drug substance by deagglomeration/sizing to prepare a powder having a specified particle size distribution. Furthermore, when the water content is adjusted to a value suitable for the compression into tables in the present pre-processing method, water also acts as a binder and tablets having hardness of 30 N or more can be manufactured. According to the present pre-processing method, particle of a drug substance which is present in the form of small secondary-aggregated masses can be dispersed excellently. Therefore, even when the preparation is carried out by a dry direct compression method or a wet continuous granulation system subsequent to the present pre-processing method, it becomes possible to manufacture tablets or granules having excellent content uniformly. The deagglomeration/sizing method which is the present pre-processing method can be employed for the manufacture of tablets or granules, as well as the manufacture of a powder preparation or the like.

Therefore, according to the present pre-processing method, a dispersant (e.g., Carplex, Aerosil) is blended in an amount of about 0.1 to about 3.0% by weight relative to 100% by weight of the final preparation in crystals having larger particle diameters formed in the crystallization step in the synthesis of the drug substance or secondary aggregates of the drug substance and then dispersing/making adhere the dispersant in/onto the surfaces of the particles of the drug substance using a deagglomerating/sizing machine. In this manner, the particle diameters of the mixed powder can be controlled, and the flowability and dispersibility of the mixed powder can be improved. Alternatively, it is also possible to blend a carboxyvinyl polymer, a water-soluble polymer or the like in an amount of 0.0 to 25.0% by weight and further blend microcrystalline cellulose, a sugar alcohol, a disintegrating agent or the like in an amount of 1.0 to 35.0% by weight to the mixed powder, then disperse and make adhere these components, and then feed the resultant mixture to a wet continuous granulation system. On the other hand, the content uniformity as well as the solubility of tablets can also be improved by blending microcrystalline cellulose, a disintegrating agent or the like in an amount of 0.5 to 45% by weight and a surfactant (solubilizing agent) powder in the mixed powder, then optionally adjusting the water content in the mixed powder, then further blending a water-soluble additive (e.g., a sugar alcohol, hydroxypropyl cellulose, polyvinylpyrrolidone, a carboxyvinyl polymer) in an amount of 0.0 to 35.0% by weight in the mixed powder, then dispersing/making adhere these components uniformly, and then compressing the mixed powder into tablets by a direct compression method.

As the additives to be used in the present pre-processing method, various additives that have been commonly used in the manufacture of a preparation can be blended appropriately depending on the intended use. Examples of the additives include a disintegrating agent, a binder, a flavoring agent, a coloring agent, a tensioning agent, a surfactant (solubilizing agent), an anti-oxidative agent, a preservative agent, a plasticizing agent, a pH modifier, a sweetening agent, and a fragrance.

Examples of the dispersant to be used in the present pre-processing method include hydrated silicon dioxide, light anhydrous silicic acid, synthetic aluminum silicate, heavy anhydrous silicic acid, magnesium aluminum hydroxide, magnesium aluminometasilicate, and dibasic calcium phosphate granules, and the dispersant is preferably hydrated silicon dioxide or light anhydrous silicic acid, more preferably hydrated silicon dioxide. These dispersants may be used singly, or arbitrary two or more of them may be used in combination.

The blending ratio of the dispersant in the present invention is not particularly limited, and is 0.1 to 6.0% by weight, preferably 0.3 to 2.0% by weight, relative to 100% by weight of the preparation. The dispersant does not necessarily cover entirely the surfaces of the particles of the drug substance. The particle diameter of the dispersant is preferably 1/10 or less, more preferably 1/100 or less, of the particle diameter of each of the particles of the drug substance.

In the case where a surfactant (solubilizing agent) is blended in the present pre-processing method, basically a powder solubilizing agent can be blended together with the dispersant. When it is intended to adjust the water content, it is possible to dissolve a solubilizing agent (e.g., Polysorbate 80 that has a liquid form) in water and add the resultant solution simultaneously with the adjustment of the water content. Examples of the surfactant (solubilizing agent) to be used in the present present pre-processing method include: a powdery surfactant (powdery solubilizing agent), such as a macrogol powder, e.g., macrogol 4000, macrogol 6000, or macrogol 20000, and sodium lauryl sulfate ; and a liquid surfactant (liquid solubilizing agent), such as polysorbate 20, polysorbate 40, polysorbate 80, macrogol 200, and macrogol 400. Among these, polysorbate, macrogol (polyethyleneglycol), sodium lauryl sulfate and the like which are used for improving dissolution and the like are preferable. These surfactants (solubilizing agents) may be used singly, or arbitrary two or more of them may be used in combination.

The blending ratio of the surfactant (solubilizing agent) in the present invention is not particularly limited, and is 0.0 to 6.0% by weight, preferably 0.5 to 3.0% by weight, relative to 100% by weight of the preparation.

Examples of the excipient to be used in the present pre-processing method include a sugar (e.g., lactose, glucose, fructose, sucrose), a sugar alcohol (D-mannitol), microcrystalline cellulose, powdered cellulose, corn starch, potato starch, partly pregelatinized starch, sodium carboxymethyl starch, dextrin, β-cyclodextrin, carmellose sodium, light anhydrous silicic acid, hydrated silicon dioxide, silicon dioxide, precipitated calcium carbonate, anhydrous dibasic calcium phosphate, magnesium oxide, titanium oxide, calcium lactate, magnesium aluminate metasilicate, synthetic hydrotalcite, talc, and kaolin, preferably microcrystalline cellulose. These excipients may be used singly, or arbitrary two or more of them may be used in combination.

The blending ratio of the excipient in the present invention is not particularly limited, and is 0.0 to 85.0% by weight, preferably 2.0 to 60.0% by weight, relative to 100% by weight of the preparation.

Examples of the disintegrating agent to be used in the present pre-processing method include carboxymethylcellulose (e.g., carmellose, carmellose sodium, carmellose calcium, croscarmellose sodium, microcrystalline cellulose-carmellose sodium), carboxymethyl starch (e.g., carboxymethyl starch, sodium carboxymethyl starch (e.g., sodium starch glycolate)), crospovidone, low-substituted hydroxypropylcellulose, low-substituted sodium hydroxymethyl starch, starch (e.g., partly pregelatinized starch, corn starch, potato starch), alginic acid, and bentonite. The disintegrating agent is preferably crospovidone, low-substituted hydroxypropylcellulose, sodium carboxymethyl starch, or partly pregelatinized starch, more preferably crospovidone or low-substituted hydroxypropylcellulose, particularly preferably low-substituted hydroxypropylcellulose. These disintegrating agents may be used singly, or arbitrary two or more of them may be used in combination.

The blending ratio of the disintegrating agent in the present invention is not particularly limited, and is 0.0 to 30.0% by weight, preferably 1.5 to 20.0% by weight, relative to 100% by weight of preparation.

The sustained-release base material to be used in the present pre-processing method is preferably one which, when contacting with water, can form a hydrogel to control the release of a drug therefrom. Examples of the sustained-release base material include: a cellulose derivative such as hydroxypropylcellulose (a high-viscosity grade), methylcellulose, hypromellose (hydroxypropylmethylcellulose), carboxymethylcellulose, carboxymethylcellulose sodium, and carboxymethylethylcellulose; a carboxyvinyl polymer; and sodium alginate. The sustained-release base material is preferably hypromellose, carboxymethylcellulose sodium, or a carboxyvinyl polymer, more preferably hypromellose or a carboxyvinyl polymer. These sustained-release base materials may be used singly. Preferably a combination of at least two of these sustained-release base materials is used to adjust the preparation so as to exert desired sustained release properties.

The blending ratio of the sustained-release base material in the present invention is not particularly limited, and is 0.0 to 20.0% by weight, preferably 1.0 to 15.0% by weight, relative to 100% by weight of the preparation.

Examples of the lubricant to be used in the present pre-processing method include stearic acid, magnesium stearate, calcium stearate, talc, sucrose esters of fatty acids, glycerol esters of fatty acids, a hydrogenated oil, polyethylene glycol, dimethyl polysiloxane, carnauba wax, sodium lauryl sulfate, yellow beeswax, and white beeswax, preferably magnesium stearate. These lubricants may be used singly, or arbitrary two or more of them may be used in combination.

The blending ratio of the lubricant in the present invention is not particularly limited, and is 0.05 to 3.0% by weight, preferably 0.1 to 2.5% by weight, relative to 100% by weight of the preparation.

The binder to be used in the present pre-processing method is a liquid or powdery binder, such as a polyvinyl alcohol-acrylic acid-methyl methacrylate copolymer (trade name: POVACOAT), hydroxypropyl cellulose (HPC), hydroxypropylmethyl cellulose (HPMC), polyvinylpyrrolidone, a polyvinyl alcohol-polyethylene glycol-graft copolymer, an ethyl acrylate-methyl methacrylate copolymer and copolyvidone (trade name: Kollidon). Among these binders, a water-soluble polymer binder having a small molecular weight also has an effect to adhere to the surfaces of the particles of the drug substance to decrease a surface tension of the drug substance, and therefore can improve the solubility of a hardly soluble drug substance. A water-soluble polymer binder having a large molecular weight can form a gel upon the binding of water thereto to delay the dissolution of the drug substance, and therefore has a release sustaining effect.

The blending ratio of the binder in the present invention is not particularly limited, and is 0.1 to 5.0% by weight, preferably 0.5 to 3.0% by weight, relative to 100% by weight of the preparation. As the binder to be used in the present invention, coporidone is preferably used because the shapability can be further improved.

### [Examples]

Hereinbelow, the present invention will be described concretely with reference to examples. However, the present invention is not limited by the following examples in any way.

The particle size distribution measurement machine used was a ro-tap-type sieve shaker (Iida-Seisakusho Japan Corporation, Japanese Pharmacopoeia] [Examples 1 to 10 and Comparative Examples 1 and 2] or Mastersizer 2000 (Malvern, dispersion compression air pressure: 2 to 4 bars) [Examples 11 to 14]. The deagglomerating/sizing machine used was a grinding stone-type mill Supermasscolloider MKCA6-5JR or MKZA10-15J (Masuko Sangyo Co., Ltd.) or Comil QC-197S or QC-U20 (Powrex Corporation). The tablet pressing machine used was a rotary tablet pressing machine VEL5 model (Kikusui Seisakusho Ltd.) or a tablet pressing machine HT-CVX-MS model (Hata Tekkosho Co., Ltd.). In the examples, all % (percent) are by weight (i.e., % by weight (percent by weight)), unless otherwise specified.

When an additive (e.g., microcrystalline cellulose, a disintegrating agent) is blended, the particle diameter of the additive becomes dominant. Therefore, a particle diameter is often shown as a larger value.

### Example 1

A pregabalin powder (200.0 g) that contained particles each having a particle size of 500 pm or more in an amount of 31% and particles each having a particle size of 60 µm or less in an amount of 14.5% and therefore had non-uniform particle diameters and Carplex (1.5 g) were deagglomerated/sized and uniformly dispersed using a deagglomerating/sizing machine (a grinding stone-type mill, trade name: "Supermasscolloider": Masuko Sangyo Co., Ltd.) to yield a powder. Subsequently, microcrystalline cellulose (KG-1000) (35.0 g), a surfactant (solubilizing agent) powder (2.5 g) and D-mannitol (trade name: "Mannit P", Mitsubishi Shoji Foodtech Co. Ltd.) (25.0 g) were added to the powder. The resultant mixture was deagglomerated/sized and uniformly dispersed to yield a powder for direct compression use (264.0 g). The powder was compressed into tablets using a tablet pressing machine manufactured by Kikusui Seisakusho Ltd. (VEL5 model, compression pressure: 14 kN) to yield tablets having tablet hardness of 119 N. The tablets had a dissolution rate at 15 minutes of 89%.

| [Components (blended amounts)] | [Blending ratio (% by weight)] |
|---|---|
| Pregabalin (200.0g) | 74.75% |
| (particles each having a particle size of 500 pm or more made up 31% and particles each having a particle size of 60 µm or less made up 14.5% in 100% | |
| of pregabalin) | |
| Carplex (1.5g) | 0.56% |
| Deagglomeration/sizing 1 (Supermasscolloider) | |
| (particles each having a particle size of 180 pm or more made up 12.5%, and fine particles each having a particle size of 60 pm or less made up 1.5% in 100% of the powder given after the deagglomeration/sizing 1) | |
| Microcrystalline cellulose (KG-1000) (135.0g) | 13.25% |
| Macrogol 4000 (2.5g) | 0.94% |
| Mannit P (25.0g) | 9.47% |
| Deagglomeration/sizing 2 (Comil) | |
| (particles each having a particle size of 180 pm or more made up 16.1%, and fine particles each having a particle size of 60 µm or less made up 9.5% in 100% of the powder given after the deagglomeration/sizing 2) | |

### Example 2

A pregabalin powder (300.0 g), which contained particles each having a particle size of 500 µm or more in an amount of 31% and particles each having a particle size of 60 µm or less in an amount of 14.5% and therefore had non-uniform particle diameters, and Carplex (1.6 g) were deagglomerated/sized and uniformly dispersed using a deagglomerating/sizing machine (Supermasscolloider), then a disintegrating agent (L-HPC) (5.0 g), lactose hydrate (22.0 g) and a Macrogol powder (5.5 g) were added to the resultant product, and the resultant mixture was deagglomerated/sized and uniformly dispersed to yield a powder (334.1 g). The powder was compressed into tablets using a tablet pressing machine manufactured by Kikusui Seisakusho Ltd. (VEL5 model, compression pressure: 14 kN) to yield tablets having tablet hardness of 117 N. The tablets had a dissolution rate at 15 minutes of 89%.

| [Components (blended amounts)] | [Blending ratio (% by weight)] |
|---|---|
| Pregabalin (300.0g) | 89.8% |
| (particles each having a particle size of 500 pm or more made up 31% and particles each having a particle size of 60 pm or less made up 14.5% in 100% of pregabalin) | |
| Carplex (1.6g) | 0.5% |
| Deagglomeration/sizing 1 (Supermasscolloider) | |
| (particles each having a particle size of 180 pm or more made up 9.8%, and fine particles each having a particle size of 60 pm or less made up 3.5% in 100% of the powder given after the deagglomeration/sizing 1) | |
| Lactose hydrate (22.0g) | 6.5% |
| Disintegrating agent (L-HPC) (5.0g) | 1.5% |
| Macrogol 6000 (5.5g) | 1.6% |
| Deagglomeration/sizing 2 (Comil) | |
| (particles each having a particle size of 180 pm or more made up 7.2 and fine particles each having a particle size of 60 pm or less made up 12.4% in 100% of the powder given after the deagglomeration/sizing 2) | |

### Example 3

Carplex (1.5 g) and a disintegrating agent (L-HPC) (15.0 g) were added to a celecoxib powder (200.0 g) which contained particles each having a particle size of 500 pm or more in an amount of 32.1% and particles each having a particle size of 60 µm or less in an amount of 18.5% and therefore had non-uniform particle diameters, and the resultant mixture was deagglomerated/sized and uniformly dispersed using a deagglomerating/sizing machine (Supermasscolloider) to yield a powder. Subsequently, lactose hydrate (48.0 g), a Macrogol 4000 powder (5.5 g), HPC-SSL (9.0 g) and a lubricant (2.0 g) were added to the powder, and the resultant mixture was deagglomerated/sized and uniformly dispersed to yield a powder for direct compression use (281.0 g). The powder was compressed into tablets using a tablet pressing machine manufactured by Kikusui Seisakusho Ltd. (VEL5 model, compression pressure: 14 kN) to yield tablets having tablet hardness of 121 N. The tablets had a dissolution rate at 15 minutes of 91%.

| [Components (blended amounts)] | [Blending ratio (% by weight)] |
|---|---|
| Celecoxib (200.0g) | 71.2% |
| (particles each having a particle size of 500 µm or more made up 32.1% and particles each having a particle size of 60 µm or less made up 18.5% in 100% of celecoxib) | |
| Disintegrating agent (L-HPC) (15.0g) | 5.3% |
| Carplex (1.5g) | 0.5% |
| Deagglomeration/sizing 1 (Supermasscolloider) | |
| (particles each having a particle size of 180 µm or more made up 4.9% and fine particles each having a particle size of 60 µm or less made up 13.9% in 100% of the powder given after the deagglomeration/sizing 1) | |
| Lactose hydrate (48.0g) | 17.0% |
| Macrogol 4000 powder (5.5g) | 1.9% |
| HPC-SSL (9.0g) | 3.2% |
| Lubricant (2.0g) | 0.7% |
| Deagglomeration/sizing 2 (Comil) | |
| (particles each having a particle size of 180 µm or more made up 5.5% and fine particles each having a particle size of 60 pm or less made up 15.5% in 100% of the powder given after the deagglomeration/sizing 2) | |

### Example 4

Carplex (0.3 g) was added to acetaminophen (60.0 g) which contained particles each having a particle size of 500 pm or more in an amount of 14% and particles each having a particle size of 60 pm or less in an amount of 32.9%, the resultant mixture was deagglomerated/sized and uniformly dispersed using a deagglomerating/sizing machine (Supermasscolloider), and then microcrystalline cellulose (CEOLUS KG-1000) (400.0 g) and a disintegrating agent (low-substituted hydroxypropylcellulose: L-HPC) (130.0 g) were added to the powder. Subsequently, water was added to the mixture in an amount of 1.8% relative to 100% of tablets to be manufactured to adjust the water content to a value suitable for pressing into tablets, then the resultant mixture was deagglomerated/sized and uniformly dispersed with Comil, and then PVACOAT (13.0 g) and Parteck M (120.0 g) were added to the powder. Subsequently, the mixture was deagglomerated/sized and uniformly dispersed using a deagglomerating/sizing machine (Comil, Powrex Corporation) to yield a powder (723.30 g) to be fed to a tablet pressing machine. The powder was compressed into tablets using a tablet pressing machine manufactured by Kikusui Seisakusho Ltd. (VEL5 model, compression pressure: 14 kN) to yield tablets having tablet hardness of 64.5 N. The tablets had a dissolution rate at 15 minutes of 88%.

| [Components (blended amounts)] | [Blending ratio (% by weight)] |
|---|---|
| Acetaminophen (60.0g) | 8.30% |
| (particles each having a particle size of 500 pm or more made up 14% and particles each having a particle size of 60 pm or less made up 32.9% in 100% of acetaminophen) | |
| Carplex (0.3g) | 0.04% |
| Deagglomeration/sizing 1 (Supermasscolloider) | |
| (particles each having a particle size of 180 pm or more made up 4.9% and fine particles each having a particle size of 60 pm or less made up 9.5% in 100% of the powder given after the deagglomeration/sizing 1) | |
| Microcrystalline cellulose (KG-1000) (400.0 | g) 55.30% |
| L-HPC (Disintegrating agent) (130.0g) | 17.98% |
| Water content adjustment | |
| POVACOAT (13.0g) | 1.80% |
| Parteck M (120.0g) | 16.59% |
| Deagglomeration/sizing 2 (Comil) | |
| (particles each having a particle size of 180 pm or more made up 4.5% and fine particles each having a particle size of 60 pm or less made up 17.5% in 100% of a powder given after the deagglomeration/sizing 2) | |

### Example 5

An acetaminophen powder (96.0 g) which contained particles each having a particle size of 500 µm or more in an amount of 14% and particles each having a particle size of 60 pm or less in an amount of 32.9% and Aerosil (0.4 g) were deagglomerated/sized and uniformly dispersed using a deagglomerating/sizing machine (Supermasscolloider) to yield a powder. Subsequently, microcrystalline cellulose (CEOLUS KG-1000; Asahi Kasei Chemicals Corporation) (300.0 g) and a carboxyvinyl polymer (160.0 g) were added to the powder, and resultant mixture was deagglomerated/sized and uniformly dispersed to yield a powder (556.4 g) to be fed to a wet continuous granulation system. The powder was compressed into tablets using a tablet pressing machine manufactured by Kikusui Seisakusho Ltd. (VEL5 model, compression pressure: 14 kN) to yield tablets having tablet hardness of 52 N. The tablets had a dissolution rate at 15 minutes of 92%.

| [Components (blended amounts)] | [Blending ratio (% by weight)] |
|---|---|
| Acetaminophen (96.0 g) | 17.27% |
| (particles each having a particle size of 500 pm or more made up 14% and particles each having a particle size of 60 µm or less made up 32.9% in 100% of acetaminophen) | |
| Aerosil (0.4g) | 0.07% |
| Deagglomeration/sizing 1 (Supermasscolloider) | |
| (particles each having a particle size of 180 µm or more made up 5.8% and fine particles each having a particle size of 60 µm or less made up 9.3% in 100% of the powder given after the deagglomeration/sizing 1) | |
| Microcrystalline cellulose (KG-1000) (300.0 g) | 53.92% |
| A carboxyvinyl polymer (160.0g) | 28.76% |
| Deagglomeration/sizing 2 (Comil) | |
| (particles each having a particle size of 180 µm or more made up 5.9% and fine particles each having a particle size of 60 µm or less made up 13.5% in 100% of the powder given after the deagglomeration/sizing 2) | |

### Example 6

An acetaminophen powder (700.0 g) which contained particles each having a particle size of 500 pm or more in an amount of 14% and particles each having a particle size of 60 pm or less in an amount of 32.9% and Carplex (3.2 g) were added to water to adjust the water content of the resultant mixture. Microcrystalline cellulose (KG-1000) (18.0 g) and a disintegrating agent (NBD-21) (20.0 g) were added to the mixture, and the resultant mixture was deagglomerated/sized and uniformly dispersed using a deagglomerating/sizing machine (Supermasscolloider) to yield a powder. A lubricant was blended to the powder, and the resultant mixture was deagglomerated/sized and uniformly dispersed to yield a powder for direct compression use (743.2 g). The powder was compressed into tablets using a tablet pressing machine manufactured by Kikusui Seisakusho Ltd. (VEL5 model, compression pressure: 14 kN) to yield tablets having tablet hardness of 49 N. The tablets had a dissolution rate at 15 minutes of 89.5%.

| [Components (blended amounts)] | [Blending ratio (% by weight)] |
|---|---|
| Acetaminophen (700.0 g) | 94.19% |
| (particles each having a particle size of 500 pm or more made up 14% and particles each having a particle size of 60 µm or less made up 32.9% in 100% of acetaminophen) | |
| Carplex (3.2g) | 0.43% |
| Water content adjustment | |
| Microcrystalline cellulose (KG-1000) (18.0g) | 2.42% |
| Disintegrating agent (NBD-21) (20.0g) | 2.69% |
| Deagglomeration/sizing 1 (Supermasscolloider) | |
| (particles each having a particle size of 180 µm or more made up 5.8% and fine particles each having a particle size of 60 µm or less made up 9.3% in 100% of the powder given after the deagglomeration/sizing 1) | |
| Lubricant (2.0g) | 0.27% |
| Deagglomeration/sizing 2 (Comil) | |
| (particles each having a particle size of 180 pm or more made up 5.8% and fine particles each having a particle size of 60 µm or less made up 14.3% in 100% of a powder given after the deagglomeration/sizing 2) | |

### Example 7

An acetaminophen powder (300.0 g) that contained particles each having a particle size of 500 µm or more in an amount of 14% and particles each having a particle size of 60 pm or less in an amount of 32.9% and Carplex (1.5 g) were deagglomerated/sized and uniformly dispersed using a deagglomerating/sizing machine (Supermasscolloider) to yield a powder. The water content in the powder was adjusted to a value suitable for the pressing into tables, then a carboxyvinyl polymer (55.0 g) was added to the powder, and the resultant mixture was deagglomerated/sized and uniformly dispersed. Hydroxypropyl cellulose (HPC) (7.0 g) and trehalose (3.0 g) were added to the resultant powder, the resultant mixture was deagglomerated/sized and uniformly dispersed, and a lubricant (magnesium stearate) (8.5 g) was blended to the powder to yield a powder for direct compression use (375.0 g). The powder was compressed into tablets using a tablet pressing machine manufactured by Kikusui Seisakusho Ltd. (VEL5 model, compression pressure: 14 kN) to yield tablets having tablet hardness of 49 N. The tablets had a dissolution rate at 15 minutes of 91.2%.

| [Components (blended amounts)] | [Blending ratio (% by weight)] |
|---|---|
| Acetaminophen (300.0g) | 80.0% |
| (particles each having a particle size of 500 pm or more made up 14% and particles each having a particle size of 60 pm or less made up 32.9% in 100% of acetaminophen) | |
| Carplex (1.5g) | 0.4% |
| Deagglomeration/sizing 1 (Supermasscolloider) | |
| (particles each having a particle size of 180 pm or more made up 5.8% and fine particles each having a particle size of 60 pm or less made up 9.3% in 100% of the powder given after the deagglomeration/sizing 1) | |
| Water content adjustment | |
| A carboxyvinyl polymer (55.0g) | 14.7% |
| HPC (SSL) (7.0g) | 1.9% |
| Trehalose (3.0g) | 0.8% |
| Deagglomeration/sizing 2 (Comil) | |
| (particles each having a particle size of 180 pm or more made up 6.1% and fine particles each having a particle size of 60 µm or less made up 16.3% in 100% of the powder given after the deagglomeration/sizing 2) | |
| Lubricant (8.5g) | 2.3% |

### Example 8

Aerosil (3.0 g) was added to an acetaminophen powder (200.0 g) which contained particles each having a particle size of 500 pm or more in an amount of 14% and particles each having a particle size of 60 µm or less in an amount of 32.9%, and the resultant mixture was deagglomerated/sized and uniformly dispersed using a deagglomerating/sizing machine (Supermasscolloider) to yield a powder. Microcrystalline cellulose (CEOLUS KG-1000) (350.0 g) and trehalose (130.0 g) were added to the powder, and the resultant mixture was deagglomerated/sized and uniformly dispersed to yield a powder for direct compression use (683.0 g). The powder was compressed into tablets using a tablet pressing machine manufactured by Kikusui Seisakusho Ltd. (VEL5 model, compression pressure: 14 kN) to yield tablets having tablet hardness of 51 N. The tablets had a dissolution rate at 15 minutes of 91%.

| [Components (blended amounts)] | [Blending ratio (% by weight)] |
|---|---|
| Acetaminophen (200.0g) | 29.29% |
| (particles each having a particle size of 500 pm or more made up 14% and particles each having a particle size of 60 pm or less made up 32.9% in 100% of acetaminophen) | |
| Aerosil (3.0g) | 0.44% |
| Deagglomeration/sizing 1 (Supermasscolloider) (particles each having a particle size of 180 pm or more made up 6.8% and fine particles each having a particle size of 60 pm or less made up 9.9% in 100% of the powder given after the deagglomeration/sizing 1) | |
| Microcrystalline cellulose (KG-1000) (350.0 | g) 51.24% |
| Trehalose (130.0g) | 19.03% |
| Deagglomeration/sizing 2 (Comil) | |
| (particles each having a particle size of 180 µm or more made up 5.8% and fine particles each having a particle size of 60 pm or less made up 19.3% in 100% of the powder given after the deagglomeration/sizing 2) | |

### Example 9

Carplex (3.0 g) was added to an ibuprofen powder (300.0 g) which contained particles each having a particle size of 500 pm or more in an amount of 11% and particles each having a particle size of 60 pm or less in an amount of 27.2%, and the resultant mixture was deagglomerated/sized and uniformly dispersed using a deagglomerating/sizing machine (Comil). Subsequently, a Macrogol 4000 powder (11.0 g) and microcrystalline cellulose (KG-1000) (350.0 g) were added and then HPC-SSL (26.0 g) was further added to the powder, and the resultant mixture was deagglomerated/sized and uniformly dispersed using a deagglomerating/sizing machine (Comil) to yield a powder (690.0 g). The powder was compressed into tablets using a tablet pressing machine manufactured by Kikusui Seisakusho Ltd. (VEL5 model, compression pressure: 14 kN) to yield tablets having tablet hardness of 51 N. The tablets had a dissolution rate at 15 minutes of 87.5%.

| [Components (blended amounts)] | [Blending ratio (% by weight)] |
|---|---|
| Ibuprofen (300.0g) | 43.48% |
| (particles each having a particle size of 500 pm or more made up 11% and particles each having a particle size of 60 µm or less made up 27.2% in 100% | |
| of ibuprofen) | |
| Carplex (3.0g) | 0.43% |
| Deagglomeration/sizing 1 (Comil) | |
| (particles each having a particle size of 180 µm or more made up 5.1% and fine particles each having a particle size of 60 µm or less made up 13.3% in 100% of the powder given after the deagglomeration/sizing 1) | |
| Macrogol 4000 (11.0g) | 1.59% |
| Microcrystalline cellulose (KG-1000) (350.0g) | 50.72% |
| HPC-SSL (26.0g) | 3.77% |
| Deagglomeration/sizing 2 (Comil) | |
| (particles each having a particle size of 180 pm or more made up 5.8% and fine particles each having a particle size of 60 pm or less made up 15.9% in 100% of the powder given after the deagglomeration/sizing 2) | |

### Example 10

Microcrystalline cellulose (CEOLUS KG-1000) (6.20 g) and a disintegrating agent (low-substituted hydroxypropylcellulose: L-HPC) (16.40 g) were added to an ibuprofen powder (300.0 g) which contained particles each having a particle size of 500 pm or more in an amount of 11% and particles each having a particle size of 60 µm or less in an amount of 27.2%, and the resultant mixture was agitated together. The water content of the mixture was adjusted to a value suitable for the pressing into tablets, then Carplex (2.25 g) was added to the mixture, the resultant mixture was agitated together, and the resultant mixture was deagglomerated/sized and uniformly dispersed using a deagglomerating/sizing machine (Comil) to yield a powder for direct compression use. A lubricant (magnesium stearate) (1.0 g) was blended in the powder, the resultant mixture was deagglomerated/sized and uniformly dispersed, and the resultant powder was compressed into tablets using a tablet pressing machine manufactured by Kikusui Seisakusho Ltd. (VEL5 model, compression pressure: 14 kN) to yield tables having tablet hardness of 54 N. The tablets had a dissolution rate at 15 minutes of 86%.

| [Components (blended amounts)] | [Blending ratio (% by weight)] |
|---|---|
| Ibuprofen (300.0g) | 92.1% |
| (particles each having a particle size of 500 pm or more made up 11% and particles each having a particle size of 60 pm or less made up 27.2% in 100% of ibuprofen) | |
| Microcrystalline cellulose (6.20g) | 1.9% |
| L-HPC (16.40g) | 5.0% |
| Water content adjustment | |
| Carplex (2.25g) | 0.7% |
| Deagglomeration/sizing 1 (Comil) | |
| (particles each having a particle size of 180 pm or more made up 4.8% and fine particles each having a particle size of 60 pm or less made up 7.5% in 100% of the powder given after the deagglomeration/sizing 1) | |
| Lubricant (1.00g) | 0.3% |
| Deagglomeration/sizing 2 (Comil) | |
| (particles each having a particle size of 180 pm or more made up 7.8% and fine particles each having a particle size of 60 pm or less made up 9.7% in 100% of the powder given after the deagglomeration/sizing 2; the particle | |
| diameters became slightly larger due to the elapse of time for dispersing the lubricant, adjusting the water content and the like.) | |

### Example 11

To acetaminophen (25,000 g) was added water (i.e., 543 g) in an amount of about 2% by weight relative to the whole amount of the powder. The resultant mixture was agitated together. Subsequently, microcrystalline cellulose (CEOLUS KG-1000) (867 g), low substituted hydroxypropyl cellulose (L-HPC NBD-021) (675 g) and copolyvidone (Kollidon VA64 Fine) (275 g) were added to the mixture, and the resultant mixture was agitated together. Subsequently, hydrated silicon dioxide (Carplex) (242 g) was added to the mixture, the resultant mixture was agitated together, and the resultant mixture was deagglomerated/sized and uniformly dispersed using a deagglomerating/sizing machine (Comil: screen diameter: 0.8 mm). Subsequently, magnesium stearate (83 g) was added to the powder, the resultant mixture was agitated together, and the resultant mixture was deagglomerated/sized and uniformly dispersed using a deagglomerating/sizing machine Comil; screen diameter: 1.6 mm). The resultant mixed powder was directly compressed into tablets using a tablet pressing machine HT-CVX-MS model (compression pressure: 15 N) to yield 300-mg tablets (hardness: 46 N, friability: 0.66%, dissolution rate at 15 minutes: 81%).

| [Components (blended amounts)] | [Blending ratio (% by weight)] |
|---|---|
| Acetaminophen (25,000 g) | 92.1% |
| (particles each having a particle size of 500 µm or more made up 4.53% by | |
| volume and particles each having a particle size of 50 µm or less made up 21.20% by volume in 100% by volume of acetaminophen) | |
| Water content adjustment | |
| Microcrystalline cellulose (867g) | 3.2% |
| L-HPC (675g) | 2.5% |
| Copolyvidone (275g) | 1.0% |
| Hydrated silicon dioxide (242g) | 0.9% |
| Deagglomeration/sizing 1 (Comil) | |
| Magnesium stearate (83g) | 0.3% |
| Deagglomeration/sizing 2 (Comil) | |
| (particles each having a particle size of 200 pm or more made up 31.50 % by volume and particles each having a particle size of 50 pm or less made up 30.39% by volume in 100% by volume of the powder given after the deagglomeration/sizing 2) | |

### Example 12

Hydrated silicon dioxide (Carplex) (250 g) was added to acetaminophen (25,000 g), the resultant mixture was agitated together, and the mixture was deagglomerated/sized and uniformly dispersed using a deagglomerating/sizing machine (Supermasscolloider; clearance: 1500 pm). Water (i.e., 537 g) was added to the resultant powder in an amount of about 2% by weight relative to the whole amount of the powder, the resultant mixture was agitated, and the mixture was deagglomerated/sized and uniformly dispersed using a deagglomerating/sizing machine (Comil; screen diameter: 1.6 mm). Subsequently, microcrystalline cellulose (CEOLUS KG-1000) (858 g), low substituted hydroxypropyl cellulose (L-HPC NBD-021) (668 g) and copolyvidone (Kollidon VA64 Fine) (272 g) were added to the powder, the resultant mixture was agitated together, and the mixture was deagglomerated/sized and uniformly dispersed using a deagglomerating/sizing machine (Comil: screen diameter: 1.6 mm). Magnesium stearate (83 g) was added to the powder, the resultant mixture was agitated together, and the mixture was deagglomerated/sized and uniformly dispersed using a deagglomerating/sizing machine (Comil; screen diameter: 1.6 mm). The resultant mixed powder was directly compressed into tablets using a tablet pressing machine HT-CVX-MS model (compression pressure: 15 N) to yield 300-mg tablets (hardness: 71 N, friability: 0.31%, dissolution rate at 15 minutes: 93%).

| [Components (blended amounts)] | [Blending ratio (% by weight)] |
|---|---|
| Acetaminophen (25,000 g) | 92.1% |
| (particles each having a particle size of 500 µm or more made up 4.53% by volume and particles each having a particle size of 50 µm or less made up 21.20% by volume in 100% by volume of acetaminophen) | |
| Hydrated silicon dioxide (250g) | 0.9% |
| Deagglomeration/sizing 1 (Supermasscolloider) | |
| Water content adjustment | |
| Deagglomeration/sizing 2 (Comil) | |
| Microcrystalline cellulose (858g) | 3.2% |
| L-HPC (668g) | 2.5% |
| Copolyvidone (272g) | 1.0% |
| Deagglomeration/sizing 3 (Comil) | |
| Magnesium stearate (83g) | 0.3% |
| Deagglomeration/sizing 4 (Comil) | |
| (particles each having a particle size of 200 µm or more made up 15.99% by volume and particles each having a particle size of 50 pm or less made up 45.05% by volume in 100% by volume of the powder given after the deagglomeration/sizing 4) | |

### Example 13

Hydrated silicon dioxide (Carplex) (2.9 g) was added to acetaminophen (300.0 g), the resultant mixture was agitated together, and the mixture was deagglomerated/sized and uniformly dispersed using a deagglomerating/sizing machine (Comil; screen diameter: 0.8 mm). Water (i.e., 6.5 g) was added to the resultant powder in an amount of about 2% by weight relative to the whole amount of the powder, and the resultant mixture was deagglomerated/sized and uniformly dispersed using a deagglomerating/sizing machine (Comil; screen diameter: 1.6 mm). Subsequently, microcrystalline cellulose (CEOLUS KG-1000) (10.4 g), low substituted hydroxypropyl cellulose (L-HPC NBD-021) (8.1 g) and copolyvidone (Kollidon VA64 Fine) (3.3 g) were added to the powder, and the resultant mixture was agitated together and was then deagglomerated/sized and uniformly dispersed using a deagglomerating/sizing machine (Comil: screen diameter: 1.6 mm). Magnesium stearate (1.0 g) was added to the powder, and the resultant mixture was agitated together and was then deagglomerated/sized and uniformly dispersed using a deagglomerating/sizing machine (Comil; screen diameter: 1.6 mm). The resultant mixed powder was directly compressed into tablets using a tablet pressing machine (compression pressure: 10 N) to yield 300-mg tablets (dissolution rate at 15 minutes: 77%).

| [Components (blended amounts)] | [Blending ratio (% by weight)] |
|---|---|
| Acetaminophen (300.0g) | 92.1% |
| (particles each having a particle size of 500 µm or more made up 4.53% by volume and particles each having a particle size of 50 pm or less made up 21.20% by volume in 100% by volume of acetaminophen) | |
| Hydrated silicon dioxide (2.9g) | 0.9% |
| Deagglomeration/sizing 1 (Comil) | |
| Water content adjustment | |
| Deagglomeration/sizing 2 (Comil) | |
| Microcrystalline cellulose (10.4g) | 3.2% |
| L-HPC (8.1g) | 2.5% |
| Copolyvidone (3.3g) | 1.0% |
| Deagglomeration/sizing 3 (Comil) | |
| Magnesium stearate (1.0g) | 0.3% |
| Deagglomeration/sizing 4 (Comil) | |
| (particles each having a particle size of 200 µm or more made up 36.53% by volume and particles each having a particle size of 50 µm or less made up 23.10% by volume in 100% by volume of the powder given after the deagglomeration/sizing 4) | |

### Example 14

Hydrated silicon dioxide (Carplex) (2.9 g) was added to acetaminophen (300.0 g), and the resultant mixture was agitated together and was then deagglomerated/sized and uniformly dispersed using a deagglomerating/sizing machine (Supermasscolloider; clearance: 1500 µm). Water (i.e., 6.5 g) was added to the resultant powder in an amount of about 2% by weight relative to the whole amount of the powder, and the resultant mixture was deagglomerated/sized and uniformly dispersed using a deagglomerating/sizing machine (Comil; screen diameter: 1.6 mm). Subsequently, microcrystalline cellulose (CEOLUS KG-1000) (10.4 g), low substituted hydroxypropyl cellulose (L-HPC NBD-021) (8.1 g) and copolyvidone (Kollidon VA64 Fine) (3.3 g) were added to the powder, and the resultant mixture was agitated together and was then deagglomerated/sized and uniformly dispersed using a deagglomerating/sizing machine (Comil: screen diameter: 1.6 mm). Magnesium stearate (1.0 g) was added to the powder, and the resultant mixture was agitated together and was then deagglomerated/sized and uniformly dispersed using a deagglomerating/sizing machine (Comil; screen diameter: 1.6 mm). The resultant mixed powder was directly compressed into tablets using a tablet pressing machine (compression pressure: 10 N) to yield 300-mg tablets (dissolution rate at 15 minutes: 92%).

| [Components (blended amounts)] | [Blending ratio (% by weight)] |
|---|---|
| Acetaminophen (300.0g) | 92.1% |
| (particles each having a particle size of 500 pm or more made up 4.53% by volume and particles each having a particle size of 50 pm or less made up 21.20% by volume in 100% by volume of acetaminophen) | |
| Hydrated silicon dioxide (2.9g) | 0.9% |
| Deagglomeration/sizing 1 (Supermasscolloider) | |
| Water content adjustment | |
| Deagglomeration/sizing 2 (Comil) | |
| Microcrystalline cellulose (10.4g) | 3.2% |
| L-HPC (8.1g) | 2.5% |
| Copolyvidone (3.3g) | 1.0% |
| Deagglomeration/sizing 3 (Comil) | |
| Magnesium stearate (1.0g) | 0.3% |
| Deagglomeration/sizing 4 (Comil) | |
| (particles each having a particle size of 200 pm or more made up 18.03 % by volume and particles each having a particle size of 50 pm or less made up 44.70% by volume in 100% by volume of the powder given after the deagglomeration/sizing 4) | |

In addition, with respect to each of drug substances of acetaminophen respectively having two different lot numbers which were different from those used in Examples 11 to 14, the particle size distribution was measured by the laser-diffraction method in the same manner as mentioned above. As a result, the following results were obtained: particles each having a particle size of 500 pm or more made up 3.57% by volume, and particles each having a particle size of 50 µm or less made up 25.11% by volume in 100% by volume of acetaminophen; and particles each having a particle size of 500 pm or more made up 5.82% by volume, and particles each having a particle size of 50 pm or less made up 21.08% by volume in 100% by volume of acetaminophen.

### Comparative Example 1 (comparison with Example 1)

To a pregabalin powder (200.0 g) which was non-uniform in particle size and contained particles each having a particle size of 500 µm or more in an amount of 31% or more were added Carplex (1.5 g), microcrystalline cellulose (KG-1000) (35.0 g) and a surfactant (solubilizing agent) powder (2.5 g) and then D-mannitol (trade name: Mannit P, Mitsubishi Shoji Foodtech Co. Ltd.) (22.0 g) and magnesium stearate (3.0g). The resultant mixture was agitated using a container-rotary-type mixer to yield a powder (264.0 g). The powder was compressed into tablets using a tablet pressing machine manufactured by Kikusui Seisakusho Ltd. (VEL5 model, compression pressure: 14 kN) to yield tablets having tablet hardness of 35 N. The tablets had a dissolution rate at 15 minutes of 54%.

| [Components (blended amounts)] | [Blending ratio (% by weight)] |
|---|---|
| Pregabalin (200.0g) | 75.75% |
| Carplex (1.5g) | 0.57% |
| Microcrystalline cellulose (KG-1000) (135.0g) | 13.26% |
| Macrogol 4000 (2.5g) | 0.95% |
| Mannit P (22.0g) | 8.33% |
| Magnesium stearate (3.0g) | 1.14% |
| Container-rotary-type mixer | |

### Comparative Example 2 (comparison with Example 6)

Water was added to an acetaminophen powder (700.0 g) which contained particles each having a particle size of 500 pm or more in an amount of 14% and Carplex 3.2 g) and Carplex (3.2 g) to adjust the water content in the mixture. Subsequently, microcrystalline cellulose (KG-1000) (18.0 g) and a disintegrating agent (NBD-21) (20.0 g) and a lubricant 2.0 g) were blended in the mixture, and the resultant mixture was agitated using a container-rotary-type mixer to yield a premix drug substance for direct tablet pressing use (743.2.0 g). This premix drug substance was compressed into tablets using a tablet pressing machine manufactured by Kikusui Seisakusho Ltd. (VEL5 model, compression pressure: 14 kN) to yield tablets having tablet hardness of 27 N. The tablets had a dissolution rate at 15 minutes of 65%.

| [Components (blended amounts)] | [Blending ratio (% by weight)] |
|---|---|
| Acetaminophen (700.0g) | 94.19% |
| Carplex (3.2g) | 0.43% |
| Water content adjustment | |
| Microcrystalline cellulose (KG-1000) (18.0g) | 2.42% |
| Disintegrating agent (NBD-21) (20.0g) | 2.69% |
| Lubricant (2.0g) | 0.27% |
| Container-rotary-type mixer | |

Only simple mixing steps were employed without employing a deagglomeration/sizing and dispersion step. Therefore, the particle diameters became slightly larger. However, the particle diameters were not measured.

### [Industrial Applicability]

Many of recent drug substances are hardly soluble or low flowable, and have problems about handleability during formulation, content uniformity in a preparation, tablet hardness and the like. According to the pre-processing method of the present invention, in contrast, a mixed powder comprising a drug substance and additives and having excellent flowability, solubility and uniformity can be manufactured. Therefore, the formulation becomes possible by a manufacture process such as a direct compression method, a continuous granulation system or the like which is simplified and streamline, and consequently the time or cost for the manufacture can be reduced. Therefore, the pre-processing method is very useful and practically advantageous.

## Claims

1. A pre-processing method in the manufacture of a pharmaceutical preparation, comprising: adding a dispersant and optionally other additive to a drug substance which contains particles each having a particle size of 500 µm or more in an amount of 1% by weight or more and particles each having a particle size of 60 µm or less in an amount of 10% by weight or more relative to 100% by weight of the drug substance; and then carrying out deagglomeration/sizing of the mixture to disperse and make adhere at least the dispersant and the other additive in/onto the surfaces of particles of the drug substance, thereby manufacturing a powder that contains particles each having a particle size of 180 pm or more in an amount of 25% by weight or less and particles each having a particle size of 60 µm or less in an amount of 25% by weight or less relative to 100% by weight of the powder, wherein the particle size distribution of the drug substance and the powder is measured by a sieving method.

2. A pre-processing method in the manufacture of a pharmaceutical preparation, comprising: adding a dispersant and optionally other additive to a drug substance which contains particles each having a particle size of 500 µm or more in an amount of 1% by volume or more and particles each having a particle size of 50 µm or less in an amount of 10% by volume or more relative to 100% by volume of the drug substance; and then carrying out deagglomeration/sizing of the mixture to disperse and make adhere at least the dispersant and the other additive in/onto the surfaces of particles of the drug substance, thereby manufacturing a powder that contains particles each having a particle size of 200 µm or more in an amount of 50% by volume or less and particles each having a particle size of 50 µm or less in an amount of 70% by volume or less relative to 100% by volume of the powder, wherein the particle size distribution of the drug substance and the powder is measured by a laser-diffraction method.

3. The method according to claim 1 or 2, wherein at least one of microcrystalline cellulose in an amount of 0 to 85% by weight, a disintegrating agent in an amount of 0 to 30% by weight, a surfactant (solubilizing agent) in an amount of 0 to 6% by weight, a water-soluble additive in an amount of 0 to 40% by weight and a sugar alcohol in an amount of 0 to 15% by weight each relative to 100% by weight of the preparation is blended and the resultant mixture is deagglomerated/sized at least one time to disperse and make adhere the additive in/onto the surfaces of the particles of the drug substance.

4. The method according to any one of claims 1 to 3, wherein water in an amount of 0.5 to 3.0% by weight relative to 100% by weight of the preparation is added if necessary.

5. The method according to any one of claims 1 to 4, wherein the pre-processing method is for a dry direct compression method.

6. The method according to any one of claims 1 to 4, wherein the pre-processing method is for a granulation step.

7. The method according to claim 6, wherein the granulation step is carried out in a continuous granulation system.

8. The method according to any one of claims 1 to 7, wherein the drug substance is low flowable, hardly soluble, or highly soluble but capable of forming a gel (undissolved lumps of powder).

9. The method according to any one of claims 1 to 8, wherein the drug substance is pregabalin, celecoxib, acetaminophen or ibuprofen.

10. The method according to any one of claims 1 to 9, wherein the dispersant is hydrated silicon dioxide, light anhydrous silicic acid or calcium silicate.

11. The method according to any one of claims 1 to 10, wherein the additive other than the dispersant is at least one component selected from: an aminoalkyl methacrylate copolymer E, an aminoalkyl methacrylate copolymer L, an aminoalkyl methacrylate copolymer LD, a methacrylic acid copolymer S, an ammonioalkyl methacrylate copolymer, microcrystalline cellulose, low-substituted hydroxypropylcellulose, crospovidone, light anhydrous silicic acid, hydrated silicon dioxide, calcium silicate, carboxymethyl starch sodium, titanium oxide, iron oxide, talc, starch, a lubricant; a water-soluble additive selected from a carboxyvinyl polymer, hydroxypropyl cellulose, polyvinylpyrrolidone, a polyvinyl alcohol-acrylic acid-methyl methacrylate copolymer, polyvinyl alcohol, a polyvinyl alcohol-polyethylene glycol-graft copolymer, copolyvidone, hydroxypropyl methylcellulose, lactose, a saccharide, a sugar alcohol and trehalose; and a surfactant (solubilizing agent) selected from macrogol, sodium lauryl sulfate and polysorbate.

12. The method according to any one of claims 1 to 11, wherein the deagglomeration/sizing is carried out using a grinding stone-type mill and/or a rod-shaped or impeller-type deagglomerating/sizing machine.

13. The method according to any one of claims 1 to 12, wherein each of the deagglomeration/sizing using a grinding stone-type mill and the deagglomeration/sizing using a rod-shaped or impeller-type deagglomerating/sizing machine is carried out at least one time.
